# EUROPEAN PATENT APPLICATION

(11) **EP 3 205 304 A1**
(43) Date of publication of application: **16.08.2017**
(21) Application number: 15907239.6
(22) Date of filing: 28.10.2015
(51) Int. Cl.: A61B 18/12

(54) **GRIPPING IMPLEMENT**

(71) Applicant: Olympus Corporation, Hachioji-shi, Tokyo 192-8507 (JP)
(72) Inventor: TAKEI, Yusuke, Hachioji-shi, Tokyo 192-8507 (JP)
(74) Representative: Schicker, Silvia
(86) International application number: PCT/JP2015/080365
(87) International publication number: WO 2017/072875

(57) **Abstract**

A grasping treatment instrument includes a first grasping piece, and a second grasping piece opening or closing relative to the first grasping piece by rotating relative to a base member. The second grasping piece includes a supported portion supported by a distal portion of the base member and forming a rotational axis of the second grasping piece relative to the base member, and a ring portion in which the supported portion is formed and which is formed in a ring shape continuous over an entire circumference around a longitudinal axis of the base member. A driving force, rotating the second grasping piece relative to the base member, is transmitted from the movable member to the ring portion.

## Description

### Technical Field

The present invention relates to a grasping treatment instrument which can grasp a treated target between a pair of grasping pieces.

### Background Art

Patent Literature 1, Patent Literature 2 and Patent Literature 3 disclose grasping treatment instruments, each of which can grasp a treated target between a pair of grasping pieces that constitute an end effector. In each of these grasping treatment instruments, a first grasping piece, which is one of the grasping pieces, is disposed in a distal portion of a base member which extends along a longitudinal axis. In addition, a second grasping piece, which is the other of the grasping pieces, is rotatably attached to the distal portion of the base member. By the second grasping piece rotating relative to the base member, the paired grasping pieces are opened or closed. By the paired grasping pieces being closed, a treated target, such as a biological tissue, is grasped.

### Citation List

### Patent Literature

Patent Literature 1: PCT International Publication No. 2004/112844
Patent Literature 2: U.S. Patent Application Publication No. 2014/0005652
Patent Literature 3: U.S. Patent Application Publication No. 2012/0110810

### Summary of Invention

### Technical Problem

In each of the grasping treatment instruments of the above-described Patent Literature 1 to Patent Literature 3, in a cross section perpendicular to the longitudinal axis of the base member (a cross section perpendicular to the extending direction of the second grasping piece), which passes through a coupling portion of the second grasping piece to the base member, a cross-sectional shape of the second grasping piece is formed as a shape, such as a substantially U-shape, in which a discontinuous part exists around the longitudinal axis of the base member. Thus, in the second grasping piece, rigidity lowers in the coupling portion to the base member and the vicinity thereof. For example, due to a torsional torque acting in a state in which a treated target is grasped between the paired grasping pieces, the coupling portion to the base member and the vicinity thereof in the second grasping piece greatly deform. If the amount of deformation in the second grasping piece increases, the paired grasping pieces are deviated from each other in the width direction, and the grasping pieces fail to be properly engaged. Consequently, the grasping force between the paired grasping pieces lowers, and there is a possibility that energy is not properly applied to the treated target grasped between the grasping pieces and the treatment performance deteriorates.

The present invention has been made in consideration of the above problem, and the object of the invention is to provide a grasping treatment instrument in which a pair of grasping pieces are properly engaged with each other.

### Solution to Problem

To solve above mentioned problems, according to one aspect of the invention, a grasping treatment instrument including: a base member having a distal end and a proximal end, and extending along a longitudinal axis from a proximal side to a distal side; a first grasping piece disposed in a distal portion of the base member; a second grasping piece configured to open or close relative to the first grasping piece by rotating relative to the base member, the second grasping piece including a supported portion which is supported by the distal portion of the base member and which forms a rotational axis of the second grasping piece relative to the base member, and a ring portion in which the supported portion is formed and which is formed in a ring shape continuous over an entire circumference around the longitudinal axis of the base member; and a movable member configured to transmit a driving force, which rotates the second grasping piece relative to the base member, to the ring portion, wherein the base member or the movable member includes a recess portion which is engaged with the ring portion and which is provided between the ring portion and the longitudinal axis in a radial direction of the base member.

### Advantageous Effects of Invention

According to the present invention, a grasping treatment instrument, in which a pair of grasping pieces are properly engaged with each other, can be provided. Brief Description of Drawings
FIG. 1 is a schematic view illustrating a treatment system in which a grasping treatment instrument according to a first embodiment is used,
FIG. 2 is a cross-sectional view which schematically illustrates a configuration of a coupling portion between a base member and a second grasping piece and a vicinity thereof according to the first embodiment,
FIG. 3 is a cross-sectional view taken along line III-III in FIG. 2,
FIG. 4 is a perspective view which schematically illustrates a configuration of the second grasping piece according to the first embodiment,
FIG. 5 is a schematic view which describes a work of attaching the second grasping piece to the base member and a movable member according to the first embodiment,
FIG. 6 is a cross-sectional view which schematically illustrates a configuration of a coupling portion between a base member and a second grasping piece and a vicinity thereof according to a second embodiment,
FIG. 7 is a cross-sectional view taken along line VII-VII in FIG. 6,
FIG. 8 is a cross-sectional view which schematically illustrates a state in which a cutter is located in a stored position in an end effector according to a first example, by a cross section perpendicular to a width direction of the end effector,
FIG. 9 is a schematic view illustrating a state in which the cutter is located in the stored position in a first grasping piece and cutter according to the first example, the state being viewed from a second grasping piece side,
FIG. 10 is a cross-sectional view which schematically illustrates the end effector according to the first example, by a cross section perpendicular to a longitudinal axial direction,
FIG. 11 is a cross-sectional view which schematically illustrates, by a cross section perpendicular to the width direction of the end effector, a state in which the cutter is located in a foremost projecting position in the end effector according to the first example,
FIG. 12 is a schematic view illustrating a state in which the cutter is located in the foremost projecting position in the first grasping piece and cutter according to the first example, the state being viewed from the second grasping piece side,
FIG. 13 is a cross-sectional view which schematically illustrates an end effector according to a first comparative example, by a cross section perpendicular to the longitudinal axial direction,
FIG. 14 is a perspective view which schematically illustrates a second grasping piece according to a modification of the first example,
FIG. 15 is a cross-sectional view which schematically illustrates the end effector according to the modification of the first example, by a cross-section perpendicular to the width direction of the end effector,
FIG. 16 is a cross-sectional view which schematically illustrates an end effector according to a second example, by a cross section perpendicular to the longitudinal axial direction,
FIG. 17 is a cross-sectional view which schematically illustrates an end effector according to a second comparative example, by a cross section perpendicular to the longitudinal axial direction,
FIG. 18 is a cross-sectional view which schematically illustrates an end effector according to a third example, by a cross section perpendicular to the longitudinal axial direction, and
FIG. 19 is a cross-sectional view which schematically illustrates an end effector according to a third comparative example, by a cross section perpendicular to the longitudinal axial direction.

### Description of Embodiments

### (First Embodiment)

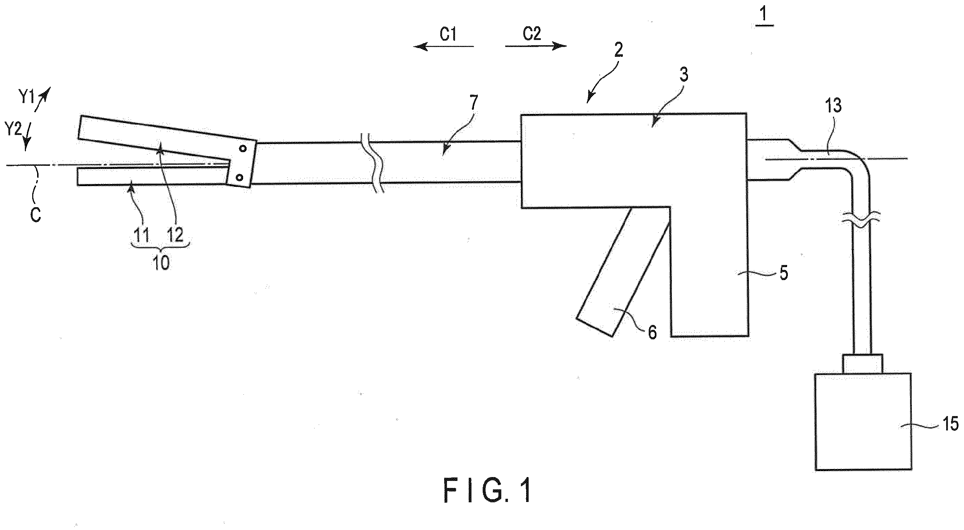

A first embodiment of the present invention will be described with reference to FIG. 1 to FIG. 5.

FIG. 1 is a view illustrating a treatment system 1 in which a grasping treatment instrument (forceps-type treatment instrument) 2 of the present embodiment is used. As illustrated in FIG. 1, the grasping treatment instrument 2 includes a housing 3 which can be held, and a base member 7. The base member 7 includes a distal end and a proximal end, and has a longitudinal axis C as a center axis. Here, a direction along the longitudinal axis C is defined as a longitudinal axial direction. In addition, one side of the longitudinal axial direction is defined as a distal side (an arrow C1 side), and the side opposite to the distal side is defined as a proximal side (an arrow C2 side). The base member 7 extends along the longitudinal axis C from the proximal side to the distal side, and the base member 7 is coupled to the distal side of the housing 3.

The housing 3 of the grasping treatment instrument 2 includes a grip (stationary handle) 5, and the grip 5 is located away from the longitudinal axis C to one side in a direction crossing (substantially perpendicular to) the longitudinal axis C. A handle (movable handle) 6 is rotatably attached to the housing 3. In the present embodiment, the handle 6 is located on the same side as the grip 5 about the longitudinal axis C, and is located on the distal side with respect to the grip 5. By the handle 6 rotating relative to the housing 3, the handle 6 opens or closes relative to the grip 5. Incidentally, the handle 6 may be provided on the proximal side with respect to the grip 5. In addition, in this embodiment, the handle 6 moves along a plane parallel to the longitudinal axis C in each of an opening motion and a closing motion relative to the grip 5, but the restriction to this is unnecessary.

One end of a cable 13 is connected to the housing 3. In addition, in the treatment system 1, the other end of the cable 13 is connected to an energy control device 15. The energy control device 15 includes a converter circuit (not shown) which converts electric power from an electric power source, such as a battery or a plug socket, to electric energy which is supplied to the grasping treatment instrument 2. The electric energy, which has been converted by the converter circuit, is output to the grasping treatment instrument 2 through an electric wiring line (not shown) or the like which extends in the inside of the cable 13. In addition, the energy control device 15 is provided with a processor which includes a CPU (Central Processing Unit) or an ASIC (Application Specific Integrated Circuit), and a storage medium such as a memory. The output of electric energy from the converter circuit is controlled by the processor. For example, by an operation input being executed by an operation button (not shown) which is attached to the housing 3, the processor detects the operation input. Thereby, electric energy is output from the converter circuit, and the electric energy is supplied to the grasping treatment instrument 2.

In addition, the grasping treatment instrument 2 is provided with an end effector 10 which treats a treated target such as a biological tissue. The end effector 10 includes a first grasping piece (first jaw) 11 which is disposed in a distal portion of the base member 7, and a second grasping piece (second jaw) 12 which is rotatably attached to the distal portion of the base member 7. By opening or closing the handle 6 relative to the grip 5, the second grasping piece 12 rotates relative to the base member 7. Thereby, the second grasping piece 12 opens or closes relative to the first grasping piece 11, and the paired grasping pieces 11 and 12 are opened or closed with respect to each other. By closing the second grasping piece 12 relative to the first grasping piece 11 in the state in which the treated target is disposed between the grasping pieces 11 and 12, the treated target is grasped between the grasping pieces 11 and 12. The opening and closing directions (a directions indicated by an arrow Y1 and an arrow Y2) of the second grasping piece 12 cross (are substantially perpendicular to) the longitudinal axial direction (a direction indicated by an arrow C1 and an arrow C2) of the base member 7. In addition, the second grasping piece 12 moves to the arrow Y1 side by the opening motion relative to the first grasping piece 11, and moves to the arrow Y2 side by the closing motion relative to the first grasping piece 11.

The first grasping piece 11 extends substantially parallel to the longitudinal axis C of the base member 7. In addition, in the state in which the second grasping piece 12 is closed relative to the first grasping piece 11, the second grasping piece 12 extends substantially parallel to the longitudinal axis C. In the meantime, the first grasping piece 11 may be attached to the distal portion of the base member 7, such as by being fixed to the distal portion of the base member 7, or may be formed as one piece with the distal portion of the base member 7. Besides, a rod member (not shown) may extend along the longitudinal axis C through the inside of the base member 7, the rod member (probe) may project to the distal side from the distal end of the base member 7, and the first grasping piece 11 may be formed by a projecting portion of the rod member from the base member 7.

In one example, an electrode (not shown) is provided in each of the paired grasping pieces 11 and 12, and electric energy (high-frequency electric energy) is supplied from the energy control device 15 to the electrode of the first grasping piece 11 and the electrode of the second grasping piece 12. By the electric energy being supplied to the electrodes of the grasping pieces 11 and 12 in the state in which the treated target is grasped, a high-frequency current flows between the electrodes through the treated target, and the treated target is treated by using the high-frequency current. In another one example, the first grasping piece 11 is formed by the above-described rod member which is provided to extend through the inside of the base member 7, and an ultrasonic transducer (not shown) including a piezoelectric element is provided in the inside of the housing 3. In this case, by electric energy being supplied from the energy control device 15 to the ultrasonic transducer, the ultrasonic transducer generates ultrasonic vibration. Then, the generated ultrasonic vibration is transmitted from the proximal side to the distal side in the rod member that is a vibration transmitting member. Thereby, the ultrasonic vibration is transmitted to the first grasping piece 11 via the rod member (vibration transmitting member), and the treated target, which is grasped between the grasping pieces 11 and 12, is treated by using the ultrasonic vibration. Furthermore, in another one example, a heating body (not shown), such as a heater, is provided in at least one of the grasping pieces 11 and 12 (end effector 10), and electric energy is supplied to the heating body from the energy control device 15. Thereby, heat is generated by the heating body, and the treated target, which is grasped between the grasping pieces 11 and 12, is treated by using the generated heat.

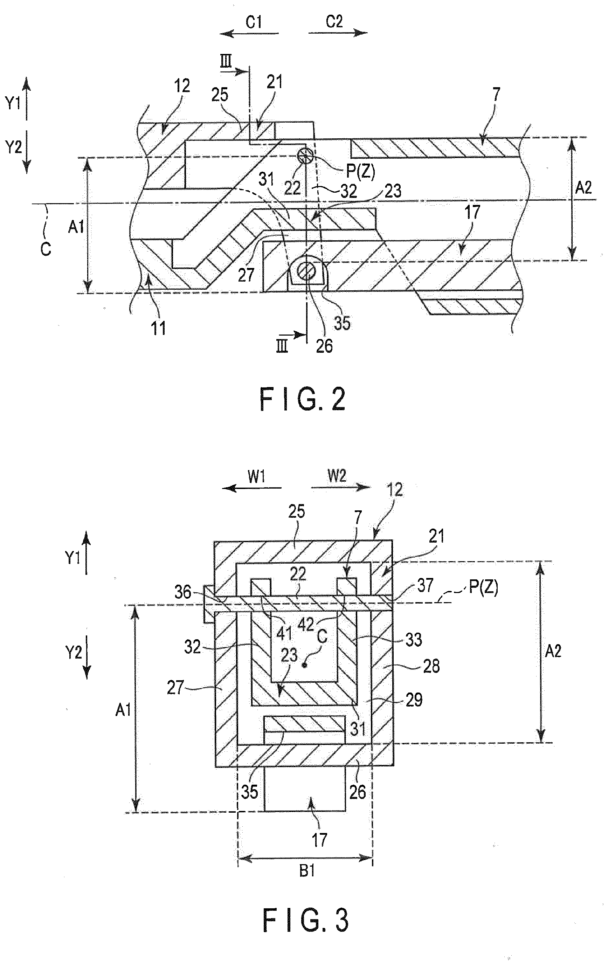

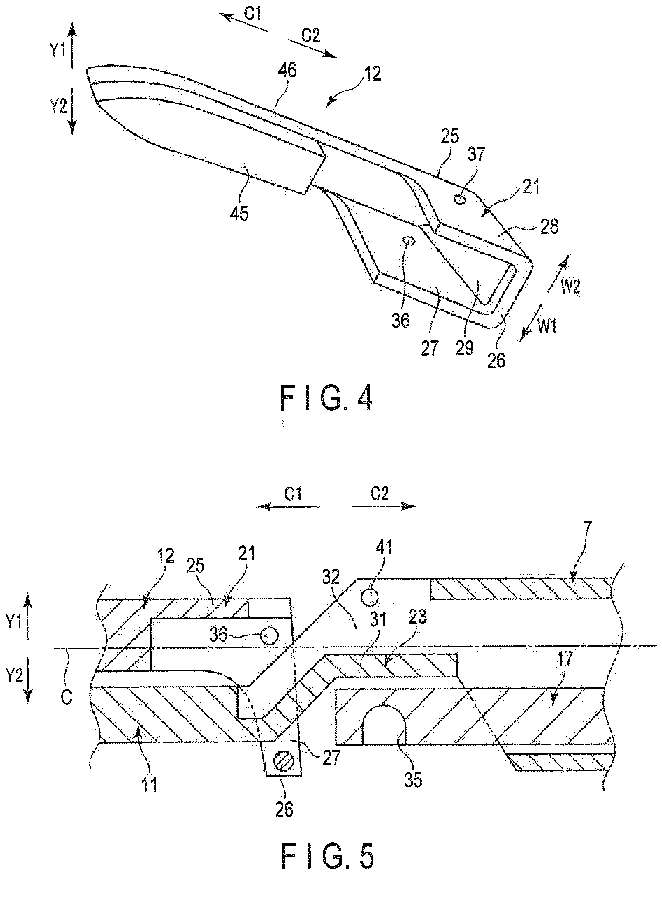

FIG. 2 is a view illustrating a configuration of a coupling portion between the base member 7 and second grasping piece 12, and a vicinity thereof. FIG. 3 is a cross-sectional view taken along line III-III in FIG. 2. FIG. 2 illustrates a cross section substantially perpendicular to a width direction (a direction indicated by an arrow W1 and an arrow W2) of the second grasping piece 12, and FIG. 3 illustrates a cross section substantially perpendicular to the longitudinal axis C of the base member 7 (an extending direction of the second grasping piece 12). Here, the width direction of the second grasping piece 12 (end effector 10) crosses (is substantially perpendicular to) the longitudinal axial direction (the direction indicated by arrow C1 and arrow C2), and crosses (is substantially perpendicular to) the opening and closing directions of the second grasping piece 12 (the directions indicated by arrow Y1 and arrow Y2). In addition, FIG. 4 is a view illustrating a configuration of the second grasping piece 12.

As illustrated in FIG. 4, the second grasping piece 12 includes a grasping surface (second grasping surface) 45 which is opposed to the first grasping piece 11, and a back surface (second back surface) 46 which faces the side opposite to the grasping surface 45. The grasping surface 45 faces the side (arrow Y2 side) toward which the second grasping piece 12 closes, and the treated target comes in contact with the grasping surface 45 in the state in which the treated target is grasped between the grasping pieces 11 and 12. In addition, the back surface 46 faces the side (arrow Y1 side) toward which the second grasping piece 12 opens.

A ring portion (annular portion) 21 is formed in a proximal portion of the second grasping piece 12. The ring portion 21 is formed of a single member as one piece, and the entirety of the ring portion 21 is one piece. In addition, the ring portion 21 is located on the proximal side with respect to the grasping surface 45. The ring portion 21 is formed in a ring shape (annular shape) which is continuous over the entire circumference around the longitudinal axis C of the base member 7 (in the circumferential direction of the second grasping piece 12), and a cavity 29 is formed on the inner peripheral side of the ring portion 21. Specifically, in the ring portion 21, there is no discontinuous part around the longitudinal axis C of the base member 7, and the cavity 29 is covered by the ring portion 21 over the entire circumference in the circumferential direction of the second grasping piece 12. Incidentally, the longitudinal axis (center axis) C of the base member 7 passes through the cavity 29 that is formed in the inner peripheral side of the ring portion 21.

The ring portion 21 is formed of a ring top wall 25, a ring bottom wall 26 and ring side walls 27 and 28. The ring top wall 25 forms a wall portion in the ring portion 21 on the side toward which the second grasping piece 12 opens, and the ring bottom wall 26 forms a wall portion in the ring portion 21 on the side toward which the second grasping piece 12 closes. In addition, the ring side wall (first ring side wall) 27 forms a wall portion in the ring portion 21 on one side (arrow W1 side) of the width direction of the second grasping piece 12, and the ring side wall (second ring side wall) 28 forms a wall portion in the ring portion 21 on the other side (arrow W2 side) of the width direction of the second grasping piece 12. A through-hole 36 is formed in the ring side wall 27 along the width direction of the second grasping piece 12, and a through-hole 37 is formed in the ring side wall 28 along the width direction of the second grasping piece 12. The through-holes 36 and 37 are located on the side (ring top wall 25 side) toward which the second grasping piece 12 opens with respect to the longitudinal axis C. In addition, although the through-holes 36 and 37 are spaced apart from each other in the width direction of the second grasping piece 12, the through-holes 36 and 37 are hardly deviated from each other (are located to substantially overlap each other) in the longitudinal axial direction of the base member 7 and in the opening and closing directions of the second grasping piece 12.

A U-shaped cross-sectional portion 23, which has a substantially U-shaped cross-sectional shape perpendicular to the longitudinal axis C, is provided in a distal portion of the base member 7. The U-shaped cross-sectional portion 23 is disposed in the cavity 29 located on the inner peripheral side of the ring portion 21, and is open to the side toward which the second grasping piece 12 opens. The U-shaped cross-sectional portion 23 is formed of a U-shaped bottom wall 31 and U-shaped side walls 32 and 33. The U-shaped bottom wall 31 forms a wall portion in the U-shaped cross-sectional portion 23 on the side toward which the second grasping piece 12 closes. In addition, the U-shaped wide wall (first U-shaped side wall) 32 forms a wall portion in the U-shaped cross-sectional portion 23 on one side of the width direction of the second grasping piece 12, and the U-shaped wide wall (second U-shaped side wall) 33 forms a wall portion in the U-shaped cross-sectional portion 23 on the other side of the width direction of the second grasping piece 12.

A through-hole 41 is formed in the U-shaped side wall 32 along the width direction of the second grasping piece 12, and a through-hole 42 is formed in the U-shaped side wall 33 along the width direction of the second grasping piece 12. The through-holes 41 and 42 are located on the side (ring top wall 25 side) toward which the second grasping piece 12 opens with respect to the longitudinal axis C. In addition, although the through-holes 41 and 42 are spaced apart from each other in the width direction of the second grasping piece 12, the through-holes 41 and 42 are hardly deviated from each other (are located to substantially overlap each other) in the longitudinal axial direction of the base member 7 and in the opening and closing directions of the second grasping piece 12. Furthermore, although the through-holes 41 and 42 are spaced apart from the through-holes 36 and 37 in the width direction of the second grasping piece 12, the through-holes 41 and 42 are hardly deviated from the through-holes 36 and 37 (are located to substantially overlap the through-holes 36 and 37) in the longitudinal axial direction of the base member 7 and in the opening and closing directions of the second grasping piece 12.

A pin member 22 is inserted through the through-holes 36, 37 of the ring side walls 27, 28 and the through-holes 41, 42 of the U-shaped side walls 32, 33. A center axis Z of the pin member 22 extends along the width direction of the second grasping piece 12. The second grasping piece 12 is supported by the distal portion of the base member 7 via the pin member 22. Accordingly, a defining surface, which defines the through-hole 36, 37 in the ring portion 21, functions as a supported portion which is supported by the distal portion of the base member 7. Thus, in the present embodiment, the supported portion (36, 37), which is formed in the ring portion 21, is located on the proximal side with respect to the grasping surface 45, and is located on the side toward which the second grasping piece 12 closes with respect to the longitudinal axis C of the base member 7.

In addition, in the present embodiment, a rotational axis (rotational center) P of the rotation of the second grasping piece 12 relative to the base member 7 is formed by the through-holes 36, 37 of the ring side walls 27, 28, the through-holes 41, 42 of the U-shaped side walls 32, 33, and the pin member 22. In the present embodiment, the rotational axis P is substantially coaxial with the center axis Z of the pin member 22, and extends in the width direction of the second grasping piece 12. In addition, the rotational axis P passes through the through-holes 36, 37, 41 and 42. Thus, in the present embodiment, the rotational axis P of the second grasping piece 12 is located on the side toward which the second grasping piece 12 opens with respect to the longitudinal axis C of the base member 7.

Besides, in the inside of the base member 7, a movable member 17 extends along the longitudinal axis C of the base member 7. A proximal portion of the movable member 17 is coupled to the handle 6 in the inside of the housing 3. In addition, a distal portion of the movable member 17 is connected to the ring portion 21 of the second grasping piece 12. By opening or closing the handle 6 relative to the grip 5, the movable member 17 moves along the longitudinal axis C relative to the base member 7. Thereby, a driving force acts on the ring portion 21 of the second grasping piece 12, and the second grasping piece 12 rotates about the rotational axis P relative to the base member 7. Specifically, by the movable member 17, the driving force, which rotates the second grasping piece 12, is transmitted to the ring portion 21.

A recess portion 35, which is formed in a recess shape, is provided in a distal portion of the movable member 17. In the distal portion of the movable member 17, the recess portion 35 is recessed from an end face on the side, toward which the second grasping piece 12 closes, to the side toward which the second grasping piece 12 opens. In addition, the recess portion 35 extends along the width direction of the second grasping piece 12, and is formed over the entire dimension (entire width) of the movable member 17 in the width direction of the second grasping piece 12. By the ring bottom wall 26 of the ring portion 21 being inserted in the recess portion 35, the ring portion 21 is engaged with the recess portion 35 of the movable member 17. Thereby, the distal portion of the movable member 17 is connected to the ring portion 21 of the second grasping piece 12. Accordingly, in the present embodiment, the recess portion 35, which is formed in the movable member 17, functions as a connection position in which the movable member 17 is connected to the ring portion 21. The connection position (35) of the movable member 17 to the ring portion 21 is located on the proximal side with respect to the grasping surface 45 of the second grasping piece 12. In this embodiment, the connection position (35) of the movable member 17 to the ring portion 21 is located on the side toward which the second grasping piece 12 closes with respect to the longitudinal axis C of the base member 7. Accordingly, the connection position (35) of the movable member 17 to the ring portion 21 is located away from the supported portion (36, 37), in which the second grasping piece 12 is supported by the base member 7, around the longitudinal axis C of the base member 7 (in the circumferential direction of the second grasping piece 12). In addition, the connection position (35) of the movable member 17 to the ring portion 21 is located on the side opposite to the supported portion (36, 37) of the second grasping piece 12 and the rotational axis P in the opening and closing directions of the second grasping piece 12, with the longitudinal axis C of the base member 7 being interposed. Incidentally, the recess portion 35 is provided between the ring portion 21 and the longitudinal axis C in the radial direction of the base member 7.

If the movable member 17 moves along the longitudinal axis C of the base member 7, the driving force, which rotates the second grasping piece 12 relative to the base member 7, acts on the connection position of the ring portion 21 to the movable member 17. Accordingly, in the ring portion 21, the ring bottom wall 26, which is engaged with the movable member 17, functions as a position of action (point of effort) where the driving force, which rotates the second grasping piece 12, acts. The position of action (26) of the driving force in the ring portion 21 is located on the proximal side with respect to the grasping surface 45 of the second grasping piece 12. In the present embodiment, the position of action (26) of the driving force in the ring portion 21 is located on the side toward which the second grasping piece 12 closes, with respect to the longitudinal axis C of the base member 7. Accordingly, the position of action (26) of the driving force in the ring portion 21 is located away from the supported portion (36, 37), in which the second grasping piece 12 is supported by the base member 7, around the longitudinal axis C of the base member 7 (in the circumferential direction of the second grasping piece 12). In addition, the position of action (26) of the driving force in the ring portion 21 is located on the side opposite to the supported portion (36, 37) of the second grasping piece 12 and the rotational axis P in the opening and closing directions of the second grasping piece 12, with the longitudinal axis C of the base member 7 being interposed. Thus, the position of action (26) of the driving force in the ring portion 21 and the supported portion (36, 37) of the second grasping piece 12 are located on opposite sides with respect to each other, with the longitudinal axis C of the base member 7 being interposed.

In the present embodiment, a reference dimension A1 in the opening and closing directions of the second grasping piece 12 is provided as a dimension from the end face on the side, toward which the second grasping piece 12 closes, in the distal portion of the movable member 17 to the rotational axis P of the second grasping piece 12 (the centers of the through-holes 41, 42 of the U-shaped cross-sectional portion 23). The reference dimension A1 agrees with a distance from an outer edge of the base member 7, which is remotest from the longitudinal axis C, to the rotational axis P in the direction crossing (substantially perpendicular to) the longitudinal axis C in the movable member 17. In addition, the cavity 29 located on the inner peripheral side of the ring portion 21 has a first cavity dimension (cavity vertical dimension) A2 in the opening and closing directions of the second grasping piece 12. The first cavity dimension A2 agrees with a distance from the ring top wall 25 to the ring bottom wall 26 in the ring portion 21. The first cavity dimension A2 agrees with a dimension of the cavity 29 located on the inner peripheral side of the ring portion 21 in the direction substantially perpendicular to the longitudinal axis C (the radial direction of the base member 7) on a plane along which the second grasping piece 12 moves relative to the first grasping piece 11. The reference dimension A1 is greater than the first cavity dimension A2. In addition, the first grasping piece 11 has a grasping piece thickness dimension L1 in the opening and closing directions of the end effector 10 (second grasping piece 12). The first cavity dimension A2 is greater than the grasping piece thickness dimension L1.

The cavity 29 has a second cavity dimension (cavity width dimension) B1 in the width direction of the end effector 10 (second grasping piece 12). The second cavity dimension B1 agrees with a distance between the ring side walls 27 and 28 in the ring portion 21. In addition, the first grasping piece 11 has a grasping piece width dimension L2 in the width direction of the end effector 10 (second grasping piece 12). The second cavity dimension B1 is greater than the grasping piece width dimension L2.

Next, the function and advantageous effects of the grasping treatment instrument 2 of the present embodiment will be described. To begin with, a description is given of the manufacture of the second grasping piece 12, and the attachment of the second grasping piece 12 to the base member 7 and movable member 17. As described above, the ring portion 21 is formed of a single member as one piece. Accordingly, a member (a main body of the second grasping piece 12) in the second grasping piece 12, which is provided with the ring portion 21, is formed as one piece by casting, metal injection molding (MIM), cutting work, etc. Since the main body of the second grasping piece 12 is formed as one piece with the ring portion 21, the number of parts of the second grasping piece 12 decreases, and the labor and cost in the manufacture of the second grasping piece 12 are reduced.

In addition, in this embodiment, the coupling portion of the second grasping piece 12 to the base member 7, and the vicinity thereof, are formed by the ring portion 21. Thus, in the coupling portion of the second grasping piece 12 to the base member 7, and the vicinity thereof, there is no discontinuous part in the second grasping piece 12 in the circumferential direction of the second grasping piece 12 (around the longitudinal axis C of the base member 7), and the ring portion 21 is continuous over the entire circumference in the circumferential direction of the second grasping piece 12. Thus, in the second grasping piece 12, rigidity increases in the coupling portion to the base member 7, and the vicinity thereof. Moreover, since the ring portion 21, which is continuous over the entire circumference in the circumferential direction of the second grasping piece 12 (around the longitudinal axis C of the base member 7), is formed of a single member as one piece, a dimensional error in manufacture of the second grasping piece 12 decreases in the coupling portion to the base member 7 and the vicinity thereof, which are formed by the ring portion 21. Thereby, the precision in dimension increases in the coupling portion of the second grasping piece 12 to the base member 7 and the vicinity thereof.

FIG. 5 is a view which describes a work of attaching the second grasping piece 12 to the base member 7 and movable member 17. As illustrated in FIG. 5, when the second grasping piece 12 is attached to the base member 7 and movable member 17, the first grasping piece 11 is inserted, from its distal side part, into the cavity 29 located on the inner peripheral side of the ring portion 21, and the base member 7 (first grasping piece 11) and movable member 17 are moved to the distal side relative to the second grasping piece 12. Here, the first cavity dimension (cavity vertical dimension) A2 of the cavity 29 is greater than the grasping piece thickness dimension L1 of the first grasping piece 11, and the second cavity dimension (cavity width dimension) B1 of the cavity 29 is greater than the grasping piece width dimension L2 of the first grasping piece 11. Thus, the first grasping piece 11 can easily be inserted into the cavity 29 (ring portion 21).

In addition, the ring bottom wall 26 of the ring portion 21 is inserted in the recess portion 35 of the movable member 17, and the ring portion 21 is engaged with the recess portion 35. Thereby, the distal portion of the movable member 17 is connected to the ring portion 21 of the second grasping piece 12. In addition, by the ring bottom wall 26 of the ring portion 21 being engaged with the recess portion 35 of the movable member 17, the through-hole (supported portion) 36, 37 of the ring portion 21 can be disposed in a position overlapping the through-hole 41, 42 of the U-shaped cross-sectional portion 23 of the base member 7 in the longitudinal direction of the base member 7 and in the opening and closing directions of the second grasping piece 12. In addition, the pin member 22 is inserted through the through-holes 36, 37, 41 and 42 in the state in which the through-hole 36, 37 of the ring portion 21 is disposed in the position overlapping the through-hole 41, 42 of the U-shaped cross-sectional portion 23 in the longitudinal direction and in the opening and closing directions of the second grasping piece 12. Thereby, the ring portion 21 is coupled to the distal portion of the base member 7, and the supported portion (36, 37) of the second grasping piece 12 (ring portion 21) is supported by the distal portion of the base member 7. Besides, the rotational axis P of the second grasping piece 12 is defined to be substantially coaxial with the center axis Z of the pin member 22.

Here, the reference dimension A1 in the opening and closing directions of the second grasping piece 12, from the end face on the side, toward which the second grasping piece 12 closes, in the distal portion of the movable member 17 to the rotational axis P of the second grasping piece 12 (the centers of the through-holes 41, 42 of the U-shaped cross-sectional portion 23), is greater than the first cavity dimension A2 of the cavity 29. Thus, in a state in which the ring portion 21 is not engaged with the recess portion 35 of the movable member 17, such as a state in which the ring portion 21 abuts on the end face on the side, toward which the second grasping piece 12 closes, in the distal portion of the movable member 17, the through-holes (supported portions) 36, 37 of the ring portion 21 cannot be disposed in the position overlapping the through-holes 41, 42 of the U-shaped cross-sectional portion 23 in the longitudinal direction of the base member 7 and in the opening and closing directions of the second grasping piece 12. Specifically, only in the state in which the ring portion 21 is engaged with the recess portion 35 of the movable member 17 (the state in which the distal portion of the movable member 17 is properly connected to the ring portion 21 of the second grasping piece 12), the through-holes (supported portions) 36, 37 of the ring portion 21 can be disposed in the position overlapping the through-holes 41, 42 of the U-shaped cross-sectional portion 23, and the ring portion 21 can be coupled to the distal portion of the base member 7. By the second grasping piece 12 being attached to the base member 7 and movable member 17 as described above, the rotational axis P is properly defined, and the second grasping piece 12 is attached to the base member 7 and movable member 17 in the positon where the driving force, which rotates the second grasping piece 12, properly acts on the ring portion 21.

Next, a treatment using the grasping treatment instrument 2 will be described. When a treatment is performed by using the grasping treatment instrument 2, the end effector 10 is inserted in a body cavity such as a peritoneal cavity. Then, a treated target, such as a biological tissue (blood vessel), is located between the grasping pieces 11 and 12. In this state, the handle 6 is closed relative to the grip 5, and the movable member 17 is moved along the longitudinal axis C. Thereby, a driving force acts on the ring bottom wall 26 of the ring portion 21, and the second grasping piece 12 rotates about the rotational axis P. Thus, the grasping pieces 11 and 12 are closed with respect to each other, and the treated target is grasped between the grasping pieces 11 and 12. At this time, the grasping surface 45 of the second grasping piece 12 comes in contact with the treated target. In the meantime, in the state in which the treated target is grasped between the grasping pieces 11 and 12, for example, based on an operation input by the operation button that is attached to the housing 3, electric energy may be output from the energy control device 15, as described above, and the treated target may be treated, as described above, by using at least one of a high-frequency current, ultrasonic vibration and heat.

In the state in which the treated target is grasped between the grasping pieces 11 and 12, there is a case in which a torsional torque acts in the circumferential direction of the second grasping piece 12. In the present embodiment, the ring portion 21 is continuous over the entire circumference in the circumferential direction of the second grasping piece 12. Thus, in the second grasping piece 12, the rigidity becomes higher in the coupling portion to the base member 7, and the vicinity thereof, than in the case of a non-annular shape. Accordingly, even if a torsional torque acts on the second grasping piece 12, deformation hardly occurs in the coupling portion to the base member 7 and the vicinity thereof in the second grasping piece, and mutual deviation between the paired grasping pieces 11 and 12 can be prevented in the width direction of the end effector 10. Thereby, even of a torsional torque acts on the second grasping piece 12, the paired grasping pieces 11 and 12 are properly engaged with each other, and the grasping force for grasping the treated target between the paired grasping pieces 11 and 12 is secured. Moreover, since the paired grasping pieces 11 and 12 are properly engaged with each other, energy such as high-frequency current is properly applied to the grasped treated target, and the treatment performance is secured.

Besides, in the present embodiment, the movable member 17 is provided with the recess portion 35 which is engaged with the ring portion 21, and the recess portion 35 is provided between the ring portion 21 and the longitudinal axis C in the radial direction of the base member 7. Thus, when the second grasping piece 12 is opened or closed, even if the distal portion of the movable member 17 deforms by receiving a force, the distal portion of the movable member 17 interferes with the ring portion 21 or first grasping piece 11. Thereby, the recess portion 35 is effectively prevented from being disengaged from the ring portion 21.

### (Second Embodiment)

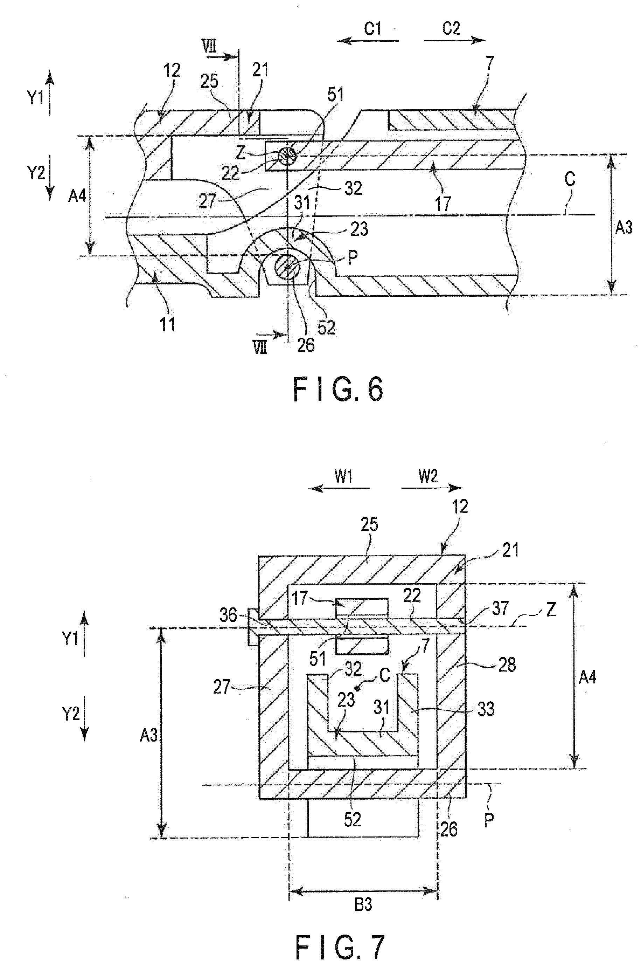

Next, a second embodiment of the present invention will be described with reference to FIG. 6 and FIG. 7. In the meantime, in the second embodiment, the configuration of the first embodiment is modified as described below. Incidentally, the same parts as in the first embodiment are denoted by like reference numerals, and a description thereof is omitted.

FIG. 6 is a view illustrating a configuration of a coupling portion between a base member 7 and a second grasping piece 12 of the present embodiment, and a vicinity thereof. FIG. 7 is a cross-sectional view taken along line VII-VII in FIG. 6. FIG. 6 illustrates a cross section substantially perpendicular to the width direction (a direction indicated by an arrow W1 and an arrow W2) of the second grasping piece 12, and FIG. 7 illustrates a cross section substantially perpendicular to the longitudinal axis C of the base member 7 (an extending direction of the second grasping piece 12).

As illustrated in FIG. 6 and FIG. 7, also in this embodiment, a U-shaped cross-sectional portion 23 is provided in a distal portion of the base member 7. The U-shaped cross-sectional portion 23 is formed of a U-shaped bottom wall 31 and U-shaped side walls 32 and 33. In the present embodiment, however, a recess portion 52, which is formed in a recess shape, is provided in the U-shaped bottom wall 31. In the U-shaped cross-sectional portion 23 (U-shaped bottom wall 31), the recess portion 52 is recessed from the end face on the side, toward which the second grasping piece 12 closes, to the side toward which the second grasping piece 12 opens. In addition, the recess portion 52 extends along the width direction of the second grasping piece 12, and is formed over the entire dimension (entire width) of the U-shaped cross-sectional portion 23 of the base member 7 in the width direction of the second grasping piece 12.

Also in this embodiment, a ring portion (annular portion) 21 is formed in the second grasping piece 12. The ring portion 21 is formed of a ring top wall 25, a ring bottom wall 26 and ring side walls 27 and 28. By the ring bottom wall 26 of the ring portion 21 being inserted in the recess portion 52, the ring portion 21 is engaged with the recess portion 52 of the base member 7. Thereby, the second grasping piece 12 is attached to the base member 7, and the second grasping piece 12 is supported by the distal portion of the base member 7. In the present embodiment, the ring bottom wall 26 of the ring portion 21 functions as a supported portion which is supported by the distal portion (recess portion 52) of the base member 7. Accordingly in this embodiment, the supported portion (26), which is formed in the ring portion 21, is located on the proximal side with respect to the grasping surface 45, and is located on the side, toward which the second grasping piece 12 closes, with respect to the longitudinal axis C of the base member 7. Incidentally, in this embodiment, too, the recess portion 52 is provided between the ring portion 21 and the longitudinal axis C in the radial direction of the base member 7.

In addition, in this embodiment, a rotational axis (rotational center) P of the rotation of the second grasping piece 12 relative to the base member 7 is formed by the recess portion 52 of the base member 7 and the ring bottom wall 26. Specifically, by the supported portion (26) being engaged with the recess portion 52 of the base member 7, the second grasping piece 12 is supported by the base member 7 in a manner to be rotatable about the rotational axis P. In this embodiment, the rotational axis P extends along the width direction of the second grasping piece 12, and the rotational axis P passes through the ring bottom wall 26 of the ring portion 21 and the recess portion 52. Thus, in the present embodiment, the rotational axis P of the second grasping piece 12 is located on the side, toward which the second grasping piece 12 closes, with respect to the longitudinal axis C of the base member 7.

In addition, in this embodiment, in place of the recess portion 35, a through-hole 51 is formed in a distal portion of the movable member 17. The through-hole 51 extends along the width direction of the second grasping piece 12. Also in this embodiment, in the ring portion 21 of the second grasping piece 12, the through-hole 36 is formed in the ring side wall 27, and the through-hole 37 is formed in the ring side wall 28. The through-holes 36, 37 and 51 are located on the side (ring top wall 25 side), toward which the second grasping piece 12 opens, with respect to the longitudinal axis C. In addition, although the through-hole 51 is spaced apart from the through-holes 36 and 37 in the width direction of the second grasping piece 12, the through-hole 51 is hardly deviated from (is located to substantially overlap) the through-holes 36 and 37 in the longitudinal axial direction of the base member 7 and in the opening and closing directions of the second grasping piece 12.

In the present embodiment, a pin member 22 extends in the width direction of the second grasping piece 12 through the through-holes 36, 37 and 51, and the pin member 22 is inserted through the through-holes 36, 37 and 51. Thereby, the distal portion of the movable member 17 is connected to the ring portion 21 of the second grasping piece 12. Accordingly, in the present embodiment, the through-hole 51, which is formed in the movable member 17, functions as a connection position at which the movable member 17 is connected to the ring portion 21. The connection position (51) of the movable member 17 to the ring portion 21 is located on the proximal side with respect to the grasping surface 45 of the second grasping piece 12. In this embodiment, the connection position (51) of the movable member 17 to the ring portion 21 is located on the side, toward which the second grasping piece 12 opens, with respect to the longitudinal axis C of the base member 7. Accordingly, the connection position (51) of the movable member 17 to the ring portion 21 is located away from the supported portion (26), at which the second grasping piece 12 is supported by the base member 7, around the longitudinal axis C of the base member 7 (in the circumferential direction of the second grasping piece 12). In addition, the connection position (51) of the movable member 17 to the ring portion 21 and the center axis Z of the pin member 22 are located on the side opposite to the supported portion (26) of the second grasping piece 12 and the rotational axis P in the opening and closing directions of the second grasping piece 12, with the longitudinal axis C of the base member 7 being interposed.

Also in this embodiment, if the movable member 17 moves along the longitudinal axis C of the base member 7, the driving force, which rotates the second grasping piece 12 relative to the base member 7, acts on the connection position of the ring portion 21 to the movable member 17. Accordingly, the through-hole 36, 37, in which the ring portion 21 is connected to the movable member 17, and a defining surface, which defines the through-hole 36, 37, function as a position of action (point of effort) where the driving force, which rotates the second grasping piece 12, acts. The position of action (36, 37) of the driving force in the ring portion 21 is located on the proximal side with respect to the grasping surface 45 of the second grasping piece 12. In the present embodiment, the position of action (36, 37) of the driving force in the ring portion 21 is located on the side, toward which the second grasping piece 12 opens, with respect to the longitudinal axis C of the base member 7. Accordingly, the position of action (36, 37) of the driving force in the ring portion 21 is located away from the supported portion (26), at which the second grasping piece 12 is supported by the base member 7, around the longitudinal axis C of the base member 7 (in the circumferential direction of the second grasping piece 12). In addition, the position of action (36, 37) of the driving force in the ring portion 21 is located on the side opposite to the supported portion (26) of the second grasping piece 12 and the rotational axis P in the opening and closing directions of the second grasping piece 12, with the longitudinal axis C of the base member 7 being interposed. Thus, in this embodiment, too, the position of action (36, 37) of the driving force in the ring portion 21 and the supported portion (26) of the second grasping piece 12 are located on opposite sides with respect to each other, with the longitudinal axis C being interposed.

In the present embodiment, a reference dimension A3 in the opening and closing directions of the second grasping piece 12 is provided as a dimension from the end face on the side, toward which the second grasping piece 12 closes, in the distal portion (U-shaped cross-sectional portion 23) of the base member 7 to the center axis Z of the pin member 22 (the center of the through-hole 51 of the movable member 17). The reference dimension A3 agrees with a distance from an outer edge, which is remotest from the longitudinal axis C, to the center axis Z in the direction crossing (substantially perpendicular to) the longitudinal axis C in the base member 17. In addition, the cavity 29 located on the inner peripheral side of the ring portion 21 has a first cavity dimension (cavity vertical dimension) A4 in the opening and closing directions of the second grasping piece 12. The first cavity dimension A4 agrees with a distance from the ring top wall 25 to the ring bottom wall 26 in the ring portion 21. The first cavity dimension A4 agrees with a dimension of the cavity 29 located on the inner peripheral side of the ring portion 21 in the direction substantially perpendicular to the longitudinal axis C (the radial direction of the base member 7) on a plane along which the second grasping piece 12 moves relative to the first grasping piece 11. The reference dimension A3 is greater than the first cavity dimension A4. In addition, the first cavity dimension A4 is greater than the grasping piece thickness dimension L1 of the first grasping piece 11 in the opening and closing directions of the end effector 10.

The cavity 29 has a second cavity dimension (cavity width dimension) B3 in the width direction of the end effector 10 (second grasping piece 12). The second cavity dimension B3 agrees with a distance between the ring side walls 27 and 28 in the ring portion 21. In addition, the second cavity dimension B3 is greater than the grasping piece width dimension L2 of the first grasping piece 11 in the width direction of the end effector 10.

Also in this embodiment, the ring portion 21 is formed of a single member as one piece. In addition, in the coupling portion of the second grasping piece 12 to the base member 7, and the vicinity thereof, there is no discontinuous part in the second grasping piece 12 in the circumferential direction of the second grasping piece 12 (around the longitudinal axis C of the base member 7), and the ring portion 21 is continuous over the entire circumference in the circumferential direction of the second grasping piece 12. Thus, like the first embodiment, the labor and cost in the manufacture of the second grasping piece 12 are reduced, and the rigidity increases in the coupling portion to the base member 7 in the second grasping piece 12, and the vicinity thereof. Moreover, like the first embodiment, the precision in dimension increases in the coupling portion of the second grasping piece 12 to the base member 7 and the vicinity thereof. Besides, in the second grasping piece 12, the rigidity becomes higher in the coupling portion to the base member 7, and the vicinity thereof, than in the case of a non-annular shape. Thus, even if a torsional torque acts on the second grasping piece 12 in the treatment in the state in which the treated target is grasped between the grasping pieces 11 and 12, the paired grasping pieces 11 and 12 are properly engaged with each other. Therefore, like the first embodiment, the grasping force for grasping the treated target between the paired grasping pieces 11 and 12 is secured, and energy is properly applied to the grasped treated target.

Also in this embodiment, when the second grasping piece 12 is attached to the base member 7 and movable member 17, the first grasping piece 11 is inserted, from its distal side part, into the cavity 29 located on the inner peripheral side of the ring portion 21, and the base member 7 (first grasping piece 11) and movable member 17 are moved to the distal side relative to the second grasping piece 12. Here, the first cavity dimension (cavity vertical dimension) A4 of the cavity 29 is greater than the grasping piece thickness dimension L1 of the first grasping piece 11, and the second cavity dimension (cavity width dimension) B3 of the cavity 29 is greater than the grasping piece width dimension L2 of the first grasping piece 11. Thus, in this embodiment, too, the first grasping piece 11 can easily be inserted into the cavity 29 (ring portion 21).

In addition, the ring bottom wall 26 of the ring portion 21 is inserted in the recess portion 52 of the base member 7 (U-shaped cross-sectional portion 23), and the ring portion 21 is engaged with the recess portion 52. Thereby, the ring portion 21 is coupled to the distal portion of the base member 7, and the supported portion (26) of the second grasping piece 12 (ring portion 21) is supported by the distal portion of the base member 7. In addition, the rotational axis P of the second grasping piece 12 is defined in the state in which the rotational axis P passes through the ring bottom wall 26 and extends along the width direction of the second grasping piece 12. Furthermore, by the ring bottom wall 26 of the ring portion 21 being engaged with the recess portion 52 of the base member 7, the through-holes (position of action of driving force) 36, 37 of the ring portion 21 can be disposed at a position overlapping the through-hole 51 of the movable member 17 in the longitudinal direction of the base member 7 and in the opening and closing directions of the second grasping piece 12. In addition, the pin member 22 is inserted through the through-holes 36, 37 and 51 in the state in which the through-holes 36, 37 of the ring portion 21 is disposed in the position overlapping the through-hole 51 of the movable member 17 in the longitudinal direction and in the opening and closing directions of the second grasping piece 12. Thereby, the distal portion of the movable member 17 is connected to the ring portion 21 of the second grasping piece 12.

Here, the reference dimension A3 in the opening and closing directions of the second grasping piece 12, from the end face on the side, toward which the second grasping piece 12 closes, in the distal portion (U-shaped cross-sectional portion 23) of the base member 7 to the center axis Z of the pin member 22 (the center of the through-hole 51 of the movable member 17), is greater than the first cavity dimension A4 of the cavity 29. Thus, in a state in which the ring portion 21 is not engaged with the recess portion 52 of the base member 7, such as a state in which the ring portion 21 abuts on the end face on the side toward which the second grasping piece 12 closes in the U-shaped cross-sectional portion 23, the through-hole (position of action of driving force) 36, 37 of the ring portion 21 cannot be disposed in the position overlapping the through-hole 51 of the movable member 17 in the longitudinal direction of the base member 7 and in the opening and closing directions of the second grasping piece 12. Specifically, only in the state in which the ring portion 21 is engaged with the recess portion 52 of the U-shaped cross-sectional portion 23 (the state in which the distal portion of the base member 7 is properly coupled to the ring portion 21 of the second grasping piece 12), the through-holes (position of action of driving force) 36, 37 of the ring portion 21 can be disposed at the position overlapping the through-hole 51 of the movable member 17, and the distal portion of the movable member 17 can be connected to the ring portion 21. By the second grasping piece 12 being attached to the base member 7 and movable member 17 as described above, the rotational axis P can properly be defined in this embodiment, too, and the second grasping piece 12 is attached to the base member 7 and movable member 17 in the positon where the driving force, which rotates the second grasping piece 12, properly acts on the ring portion 21.

### (Examples of the end effector 10 to which the above-described embodiments are applied)

Hereinafter, examples (applied examples) of the end effector 10, to which the first embodiment and second embodiment are applied, will be described.

### [First Example]

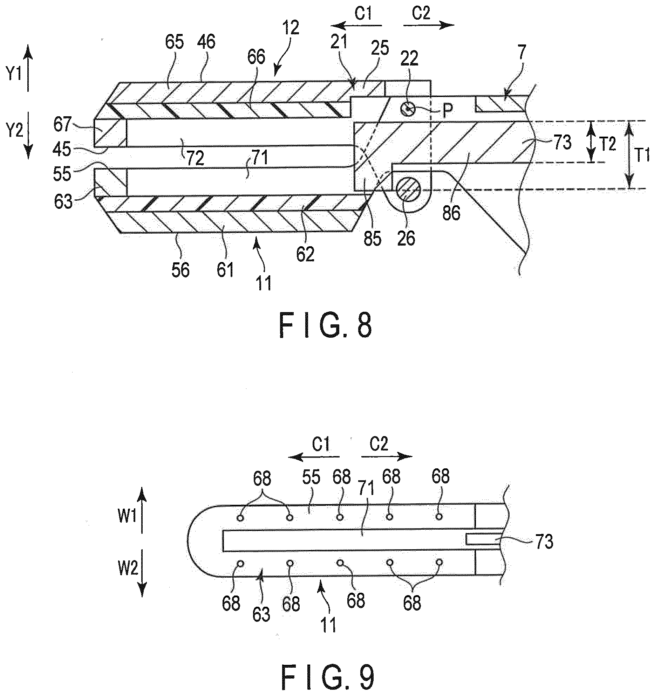

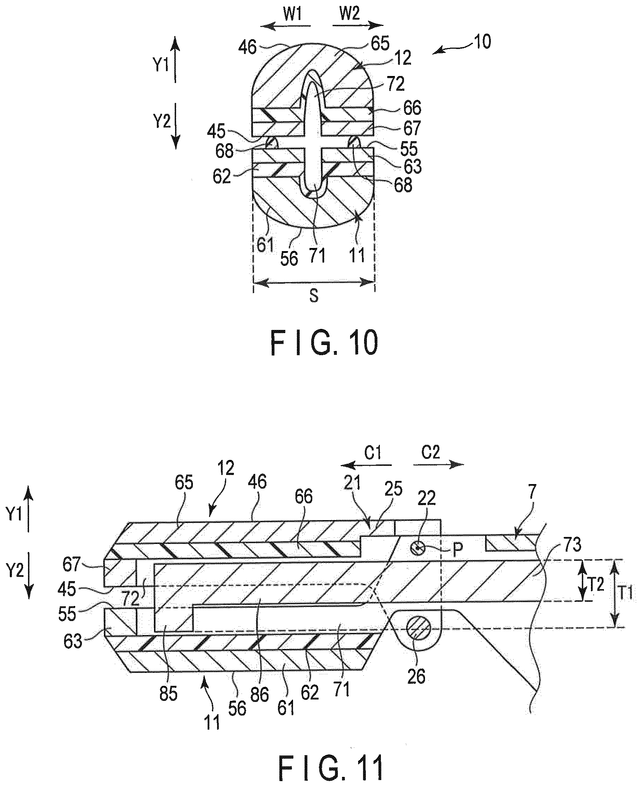

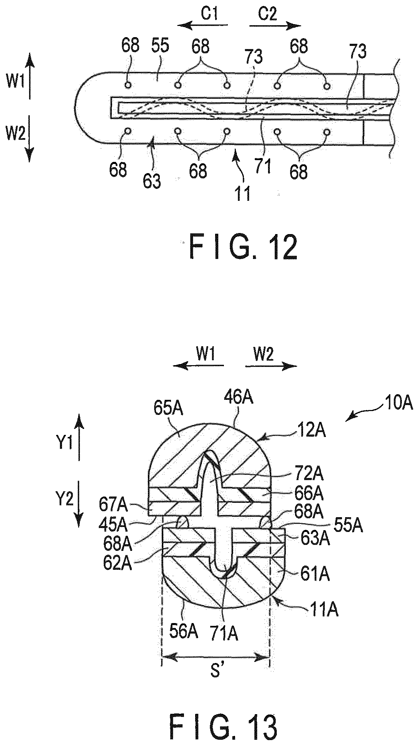

A first example will be described with reference to FIG. 8 to FIG. 12. Here, FIG. 8 and FIG. 11 illustrate an end effector 10 by a cross section perpendicular to the width direction. FIG. 9 and FIG. 12 are views illustrating the first grasping piece 11 and a cutter 73 (to be described later) as viewed from the second grasping piece 12 side. Besides, FIG. 10 illustrates the end effector 10 by a cross section perpendicular to the longitudinal axial direction of the base member 7. In addition, FIG. 8 and FIG. 9 illustrate a state in which the cutter 73 is located most proximally (at a stored position), and FIG. 11 and FIG. 12 illustrate a state in which the cutter 73 is located most distally (at a foremost projecting position).

As illustrated in FIG. 8 to FIG. 12, in the present example, the first grasping piece 11 is fixed to the base member 7. The first grasping piece 11 includes a support member (first support member) 61, an insulation member (first insulation member) 62, an electrode member (first electrode member) 63, and receiving members 68 (a plurality of receiving members 68 in this example). In the first grasping piece 11, a grasping surface (first grasping surface) 55, which is opposed to the.second grasping piece 12, is formed by the electrode member 63, and a back surface (first back surface) 56, which faces the side opposite to the grasping surface 55, is formed by the support member 61. The support member 61 is formed as one piece with the base member 7, or is attached to the base member 7 in a fixed state. The insulation member 62 is formed of an electrically insulative material, and is fixed to the support member 61 on the grasping surface 55 side. The electrode member 63 is formed of an electrically conductive material, and is fixed to the insulation member 62 on the grasping surface 55 side. In addition, the receiving members 68 are formed of an electrically insulative material, and are fixed to the electrode member 63 on the grasping surface 55. In the first grasping piece 11, a groove (first groove) 71, which is recessed from the grasping surface 55 toward the back surface 56 side, is formed by the insulation member 62 and electrode member 63. The groove 71 extends along the longitudinal axial direction.

The second grasping piece 12 is supported by the distal portion of the base member 7 via the pin member 22 like the first embodiment, and is rotatable about the rotational axis P. The second grasping piece 12 includes a support member (second support member) 65, an insulation member (second insulation member) 66, and an electrode member (second electrode member) 67. In the second grasping piece 12, a grasping surface (second grasping surface) 45, which is opposed to the first grasping piece 11, is formed by the electrode member 67, and a back surface (second back surface) 46, which faces the side opposite to the grasping surface 45, is formed by the support member 65. The support member 65 is rotatably attached to the base member 7, and the distal portion of the movable member 17 is connected to the support member 65. In addition, the above-described ring portion 21 is formed in the support member 65, and the ring portion 21 is provided with a supported portion (36, 37; 26) which is supported by the base member 7. The insulation member 66 is formed of an electrically insulative material, and is fixed to the support member 65 on the grasping surface 45 side (the side toward which the second grasping piece 12 closes). The electrode member 67 is formed of an electrically conductive material, and is fixed to the insulation member 66 on the grasping surface 45 side. In the second grasping piece 12, a groove (second groove) 72, which is recessed from the grasping surface 45 toward the back surface 46 side (the side toward which the second grasping piece 12 opens), is formed by the insulation member 66 and electrode member 67. The groove 72 extends along the longitudinal axial direction.

In the state in which the grasping pieces 11 and 12 are closed, the grasping surface 45 (electrode member 67) of the second grasping piece 12 can abut on the receiving members 68 of the first grasping piece 11. In the state in which the electrode member 67 of the second grasping piece 12 abuts on the receiving members 68 of the first grasping piece 11, the electrode member 67 of the second grasping piece 12 does not come in contact with the electrode member 63 of the first grasping piece 11.

In addition, in the present example, the cutter 73 extends along the longitudinal axis C through the inside of the base member 7. The cutter 73 is inserted in the grooves 71 and 72. The cutter 73 is movable along the longitudinal axis C relative to the base member 7 and grasping pieces 11 and 12. For example, based on an operation input by an operation lever (not shown) which is attached to the housing 3, the cutter 73 moves in the longitudinal axial direction in the inside of the base member 7 and in the grooves 71 and 72. The cutter 73 is movable between a stored position (a position illustrated in FIG. 8 and FIG. 9) which is the position disposed most proximally, and a foremost projecting position (a position illustrated FIG. 11 and FIG. 12) which is the position disposed most distally.

The cutter 73 includes a first width dimensional portion 85, and a second width dimensional portion 86 which is continuous with a proximal side of the first width dimensional portion 85. The first width dimensional portion 85 forms a distal end of the cutter 73, and is provided in only a distal portion of the cutter 73. In addition, the first width dimensional portion 85 has a first cutter width dimension T1 in the width direction of the cutter 73 (the opening and closing directions of the second grasping piece 12). The second width dimensional portion 86 forms a portion other than the first width dimensional portion 85 in the cutter 73, and forms a most part of the cutter 73. In addition, the second width dimensional portion 86 has a second cutter width dimension T2, which is less than the first cutter width dimension T1, in the width direction of the cutter 73 (the opening and closing directions of the second grasping piece 12). In the state in which the cutter 73 is located in the stored position, the first width dimensional portion 85 is located on the distal side with respect to the ring portion 21 (ring bottom wall 26) of the second grasping piece 12. Accordingly, even in the state in which the cutter 73 is located at any position (between the stored position and the foremost projecting position), the first width dimensional portion 85 is located on the distal side with respect to the ring portion 21 (ring bottom wall 26).

In the treatment in the present example, high-frequency electric energy is supplied from the energy control device 15 to the electrode members (electrodes) 63 and 67, and a high-frequency current is passed through a treated target which is grasped between the electrode members 63 and 67 (grasping surfaces 45 and 55). Thereby, the treated target is coagulated (sealed). In addition, in the present example, in addition to the treatment using the high-frequency current, the grasped treated target is cut and opened by the cutter 73 by moving the cutter 73 along the longitudinal axis C in the grooves 71 and 72.

Here, an end effector 10A of a first comparative example is illustrated in FIG. 13. Also in the end effector 10A of the first comparative example, like the first example, a first grasping piece 11A includes a support member 61A, an insulation member 62A, an electrode member 63A and receiving members 68A. A groove 71A is formed in the first grasping piece 11A. In addition, a second grasping piece 12A includes a support member 65A, an insulation member 66A, and an electrode member 67A. A groove 72A is formed in the second grasping piece 12A. In addition, a cutter 73A is movable in the grooves 71A and 72A along the longitudinal axis C. However, in the first comparative example, the second grasping piece 12A is not provided with the above-described ring portion 21. In the coupling portion of the second grasping piece 12A to the base member (not shown) and the vicinity thereof, there is a part in which the second grasping piece 12A is discontinuous around the longitudinal axis C of the base member. Thus, in the second grasping piece 12A, rigidity decreases in the coupling portion to the base member and the vicinity thereof. For example, due to a torsional torque acting in the state in which the treated target is grasped between the paired grasping pieces 11A and 12A, the coupling portion to the base member and the vicinity thereof greatly deform in the second grasping piece 12A. If the amount of deformation increases in the second grasping piece 12A, the paired grasping pieces 11A and 12A are deviated from each other, as illustrated in FIG. 13, in the width direction (direction of an arrow W1 and an arrow W2) of the end effector 10A.

By the paired grasping pieces 11A and 12A being deviated from each other in the width direction of the end effector 10A, a range S' in the width direction of the end effector 10A becomes smaller, within which a high-frequency current is passed through the treated target grasped between the electrode members 63A and 67A (grasping surfaces 45A and 55A). Consequently, a range, within which the treated target is coagulated (sealed) by the high-frequency current, becomes smaller, and the coagulation performance (sealing performance) of the treated target by the high-frequency current lowers. In addition, in the first comparative example, by the paired grasping pieces 11A and 12A being deviated from each other in the width direction of the end effector 10A, the grooves 71A and 72A are also deviated from each other in the width direction of the end effector 10A. Since the grooves 71A and 72A are deviated from each other, the amount of operational force for moving the cutter 73 along the longitudinal axis C increases, or it becomes impossible to move the cutter 73.

By contrast, in the present example, since the second grasping piece 12 is provided with the ring portion 21, the rigidity increases in the coupling portion of the second grasping piece 12 to the base member 7, and the vicinity thereof. Thus, even if a torsional torque acts on the second grasping piece 12, mutual deviation between the paired grasping pieces 11 and 12 is prevented in the width direction of the end effector 10. Since the paired grasping pieces 11 and 12 are not deviated from each other, a range S in the width direction of the end effector 10 becomes larger, within which a high-frequency current is passed through the treated target grasped between the electrode members 63 and 67 (grasping surfaces 45 and 55). Thus, the range, within which the treated target is coagulated (sealed) by the high-frequency current, becomes larger, and the coagulation performance (sealing performance) of the treated target by the high-frequency current is secured. In addition, since the paired grasping pieces 11 and 12 are not deviated from each other, the grooves 71A and 72A are not deviated from each other in the width direction of the end effector 10 (second grasping piece 12), either. Since the grooves 71 and 72 are not deviated from each other, the cutter 73 can easily be moved along the longitudinal axis C by a small amount of operational force.

In addition, in the present example, the cutter 73 is formed of the first width dimensional portion 85 and second width dimensional portion 86, and, even in the state in which the cutter 73 is located at any position, the first width dimensional portion 85 having the greater first cutter width dimension T1 is located on the distal side with respect to the ring portion 21 (ring bottom wall 26). Thus, interference between the cutter 73 and the ring portion 21 (ring bottom wall 26) is prevented, and the cutter 73 can be moved more easily along the longitudinal axis C.

### [Modification of the First Example]

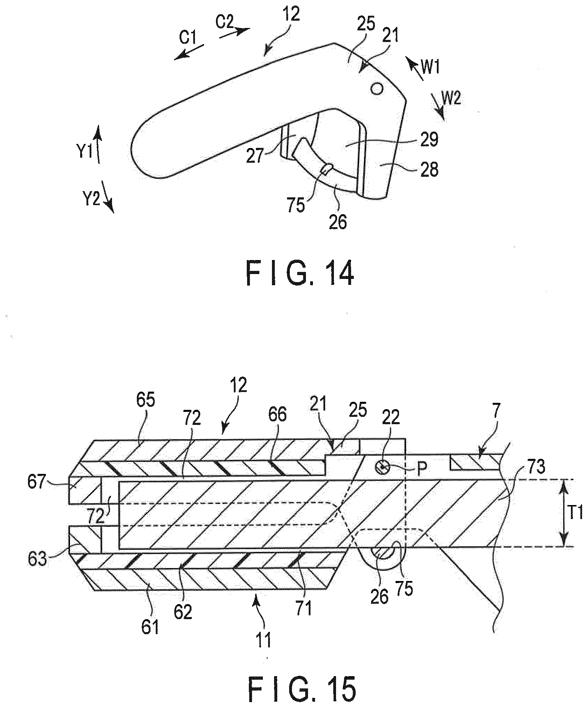

Additionally, in a modification of the first example, which is illustrated in FIG. 14 and FIG. 15, a notch (slit) 75 is formed in the ring bottom wall 26 of the ring portion 21 of the second grasping piece 12. The notch 75 is recessed on the inner peripheral surface of the ring bottom wall 26 toward the side (arrow Y2 side) toward which the second grasping piece 12 closes. In addition, in this modification, the cutter 73 has a cutter width dimension T1 in the width direction of the cutter 73 (the opening and closing directions of the second grasping piece 12) over the entire length of the cutter 73 in the longitudinal axial direction, and the cutter width dimension T1 is large over the entire length of the cutter 73 in the longitudinal axial direction. In the present modification, the cutter 73 is inserted in the notch 75 in the cavity 29 located on the inner peripheral side of the ring portion 21.

If the cutter width dimension in the width direction of the cutter 73 decreases, the cutter 73 would easily buckle in the thickness direction of the cutter 73 (the width direction of the end effector 10) (see a part indicated by a broken line in FIG. 12). In the present modification, since the cutter width dimension T1 is large over the entire length of the cutter 73 in the longitudinal axial direction, buckling of the cutter 73 in the thickness direction of the cutter 73 is prevented. Since the buckling of the cutter 73 in the thickness direction of the cutter 73 is prevented, the friction between a defining surface of the groove 71, 72 and the cutter 73 decreases. Thereby, the cutter 73 can be moved more easily along the longitudinal axis C. In addition, since the ring bottom wall 26 is provided with the notch 75, even if the cutter width dimension T1 becomes large over the entire length of the cutter 73 in the longitudinal axial direction, interference between the cutter 73 and the ring portion 21 (ring bottom wall 26) of the second grasping piece 12 can effectively be prevented.

### [Second Example]

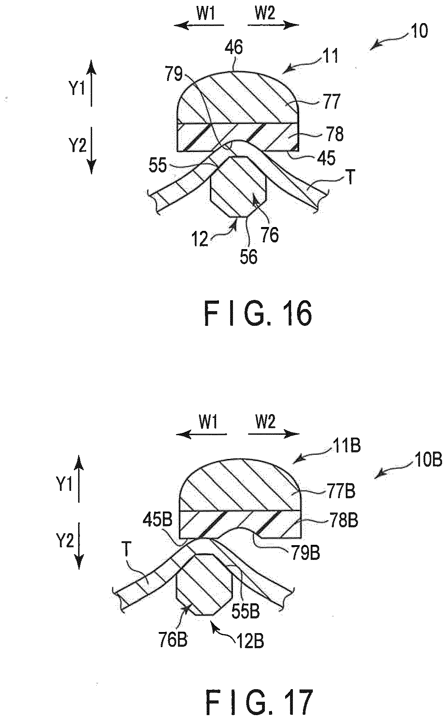

In a second example illustrated in FIG. 16, an ultrasonic transducer (not shown) is provided in the inside of the housing 3, and a rod member (probe) 76, which transmits, from the proximal side to distal side, ultrasonic vibration generated by the ultrasonic transducer, extends through the inside of the base member 7. In addition, the first grasping piece 11 is formed by a projecting portion of the rod member (vibration transmitting member) 76 to the distal side from the base member 7.

Besides, the second grasping piece 12 is rotatable about the rotational axis P relative to the base member 7, and includes a support member 77 and a pad member 78. In the second grasping piece 12, a back surface (second back surface) 46 is formed by the support member 77, and a grasping surface (second grasping surface) 45 is formed by the pad member 78. The support member 77 is rotatably attached to the base member 7, and the distal portion of the movable member 17 is connected to the support member 77. In addition, the above-described ring portion 21 is formed on the support member 77, and the ring portion 21 is provided with a supported portion (36, 37; 26) which is supported by the base member 7. The pad member 78 is formed of a resin such as PTFE (polytetrafluoroethylene), and is fixed to the support member 77 on the grasping surface 45 side. Furthermore, in the pad member 78, a receiving portion 79 is formed, which is recessed on the grasping surface 45 toward the back surface 46 side (the side toward which the second grasping piece 12 opens). By closing the grasping pieces 11 and 12, the grasping surface (first grasping surface) 55 of the first grasping piece 11 can abut on the receiving portion 79 of the pad member 78.

In the treatment in the present example, electric energy is supplied from the energy control device 15 to the ultrasonic transducer, and ultrasonic vibration is generated. Then, the generated ultrasonic vibration is transmitted to the first grasping piece 11 through the rod member 76. By the ultrasonic vibration being transmitted to the first grasping piece 11 in the state in which a treated target T is grasped between the grasping pieces 11 and 12, frictional heat occurs between the vibrating first grasping piece 11 and the treated target T, and the treated target T is coagulated (sealed) by the frictional heat.

Here, an end effector 10B of a second comparative example is illustrated in FIG. 17. Also in the end effector 10B of the second comparative example, like the second example, a first grasping piece 11B is formed by a projecting portion of a rod member 76B from the base member (not shown), and a second grasping piece 12B includes a support member 77B and a pad member 78B. However, in the second comparative example, the second grasping piece 12B is not provided with the above-described ring portion 21. In the coupling portion of the second grasping piece 12B to the base member and the vicinity thereof, there is a part in which the second grasping piece 12B is discontinuous around the longitudinal axis C of the base member. Thus, in the second grasping piece 12B, the rigidity decreases in the coupling portion to the base member and the vicinity thereof, and there is a case in which the coupling portion to the base member and the vicinity thereof greatly deform in the second grasping piece 12B. If the amount of deformation increases in the second grasping piece 12B, the paired grasping pieces 11B and 12B are deviated from each other, as illustrated in FIG. 17, in the width direction (direction of an arrow W1 and an arrow W2) of the end effector 10B.

By the paired grasping pieces 11B and 12B being displaced from each other in the width direction of the end effector 10B, the first grasping piece 11B is deviated from a receiving portion 79B of the second grasping piece 12B in the width direction of the end effector 10B. Consequently, the grasping pieces 11B and 12B are not properly engaged, and a range in the width direction of the end effector 10B decreases, within which the treated target T grasped between the grasping pieces 11B and 12B (grasping surfaces 45B and 55B) is coagulated (sealed) by the frictional heat. Thus, the coagulation performance (sealing performance) of the treated target T by the ultrasonic vibration (frictional heat) lowers.

By contrast, in the present example, since the second grasping piece 12 is provided with the ring portion 21, the rigidity increases in the coupling portion of the second grasping piece 12 to the base member 7, and the vicinity thereof. Thus, even if a torsional torque acts on the second grasping piece 12, mutual deviation between the paired grasping pieces 11 and 12 is prevented in the width direction of the end effector 10. Since the paired grasping pieces 11 and 12 are not deviated from each other, the first grasping piece 11 is not displaced from the receiving portion 79 of the second grasping piece 12 in the width direction of the end effector 10, and the grasping pieces 11 and 12 are properly engaged. Thereby, the range in the width direction of the end effector 10 increases, within which the treated target T grasped between the grasping pieces 11 and 12 (grasping surfaces 45 and 55) is coagulated (sealed) by the frictional heat. Thus, the coagulation performance (sealing performance) of the treated target T by the ultrasonic vibration (frictional heat) is secured.

### [Third Example]

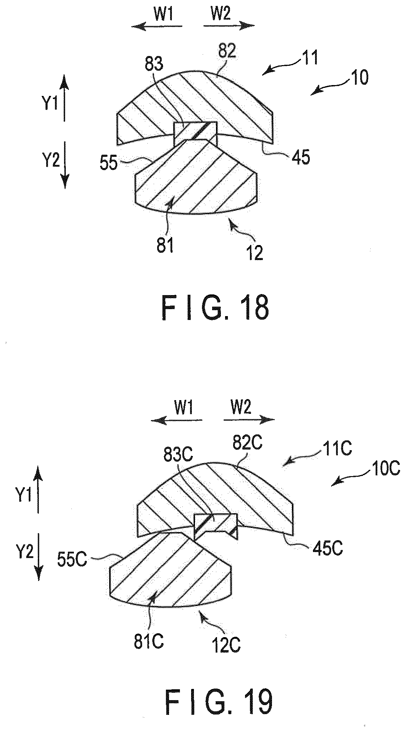

In a third example illustrated in FIG. 18, a first grasping piece 11 is formed by an electrode member (first electrode member) 81 which is fixed to the base member 7 or is formed as one piece with the base member 7. In addition, a second grasping piece 12 is rotatable about the rotational axis P relative to the base member 7, and includes an electrode member 82 and a receiving member 83. A grasping surface (second grasping surface) 45 is formed by the electrode member (second electrode member) 82 and receiving member 83. The electrode member 82 is rotatably attached to the base member 7, and the distal portion of the movable member 17 is connected to the electrode member 82. In addition, the above-described ring portion 21 is formed on the electrode member 82, and the ring portion 21 is provided with a supported portion (36, 37; 26) which is supported by the base member 7. The receiving member 83 is formed of an electrically insulative material, and is fixed to the electrode member 82 on the grasping surface 45 side. By closing the grasping pieces 11 and 12, the electrode member 81 of the first grasping surface 11 can abut on the receiving member 83 of the second grasping piece 12. In the state in which the electrode member 81 of the first grasping piece 11 abuts on the receiving member 83, the electrode members (electrodes) 81 and 82 are spaced apart from each other.

In the treatment in the present example, high-frequency electric energy is supplied from the energy control device 15 to the electrode members 81 and 82, and a high-frequency current is passed between the electrode members 81 and 82 through the treated target grasped between the grasping pieces 11 and 12. Thereby, the treated target is coagulated (sealed).

Here, an end effector 10C of a third comparative example is illustrated in FIG. 19. Also in the end effector 10C of the third comparative example, like the third example, a first grasping piece 11C includes an electrode member 81C, and a second grasping piece 12C includes an electrode member 82C and a receiving member 83C. However, in the third comparative example, the second grasping piece 12C is not provided with the above-described ring portion 21. In the coupling portion of the second grasping piece 12C to the base member (not shown) and the vicinity thereof, there is a part in which the second grasping piece 12C is discontinuous around the longitudinal axis C of the base member. Thus, in the second grasping piece 12C, the rigidity decreases in the coupling portion to the base member and the vicinity thereof, and there is a case in which the coupling portion to the base member and the vicinity thereof greatly deform in the second grasping piece 12C. If the amount of deformation increases in the second grasping piece 12C, the paired grasping pieces 11C and 12C are deviated from each other, as illustrated in FIG. 19, in the width direction (direction of an arrow W1 and an arrow W2) of the end effector 10C.

By the paired grasping pieces 11C and 12C being displaced from each other in the width direction of the end effector 10C, the electrode members 81C and 82C come in contact, short-circuit occurs in a current path of the high-frequency current, and the high-frequency current fails to be properly applied to the treated target. Consequently, the coagulation performance (sealing performance) of the treated target by the high-frequency current lowers.

By contrast, in the present example, since the second grasping piece 12 is provided with the ring portion 21, the rigidity increases in the coupling portion of the second grasping piece 12 to the base member 7, and the vicinity thereof. Thus, even if a torsional torque acts on the second grasping piece 12, mutual deviation between the paired grasping pieces 11 and 12 is prevented in the width direction of the end effector 10. Since the paired grasping pieces 11 and 12 are not deviated from each other, a mutual contact between the electrode members 81 and 82 is. prevented, and the occurrence of short-circuit in the current path of high-frequency current is prevented. Thereby, the coagulation performance (sealing performance) of the treated target by the high-frequency current is secured.

### (Other Embodiments)

In the meantime, the aspect of the end effector 10 is not restricted to the above-described configurations.

In the above-described embodiments, etc., the grasping treatment instrument (2) includes the base member (7) having the distal end and proximal end and extending along the longitudinal axis (C) from the proximal side (C2) to distal side (C1); the first grasping piece (11) disposed in the distal portion of the base member (7); and the second grasping piece (12) configured to open or close relative to the first grasping piece (11) by rotating relative to the base member (7). The second grasping piece (12) includes the supported portion (36, 37; 26) which is supported by the distal portion of the base member (7) and forms the rotational axis (P) of rotation of the second grasping piece (12) relative to the base member (7), and the ring portion (21) in which the supported portion (36, 37; 26) is formed and which is formed in the ring shape continuous over the entire circumference around the longitudinal axis (C) of the base member (7). In addition, the grasping treatment instrument (2) includes the movable member (17) configured to transmit the driving force, which rotates the second grasping piece (12) relative to the base member (7), to the ring portion (21). The base member (7) or the movable member (17) includes the recess portion (52) which is engaged with the ring portion (21) and is provided between the ring portion (21) and the longitudinal axis (C) in the radial direction of the base member (7).

Although the embodiments, etc. of the present invention have been described above, the present invention is not limited to the above-described embodiments, etc., and, needless to say, various modifications can be made without departing from the spirit of the invention.

## Claims

1. A grasping treatment instrument comprising:
a base member having a distal end and a proximal end, and extending along a longitudinal axis from a proximal side to a distal side;
a first grasping piece disposed in a distal portion of the base member;
a second grasping piece configured to open or close relative to the first grasping piece by rotating relative to the base member, the second grasping piece including a supported portion which is supported by the distal portion of the base member and which forms a rotational axis of the second grasping piece relative to the base member, and a ring portion in which the supported portion is formed and which is formed in a ring shape continuous over an entire circumference around the longitudinal axis of the base member; and
a movable member configured to transmit a driving force, which rotates the second grasping piece relative to the base member, to the ring portion,
wherein the base member or the movable member includes a recess portion which is engaged with the ring portion and which is provided between the ring portion and the longitudinal axis in a radial direction of the base member.

2. The grasping treatment instrument of Claim 1,
wherein in the ring portion, a position of action, where the driving force, which rotates the second grasping piece relative to the base member, acts, is located away from the supported portion around the longitudinal axis of the base member.

3. The grasping treatment instrument of Claim 2,
wherein in the ring portion, the supported portion and the position of action of the driving force are located on opposite sides, with the longitudinal axis of the base member being interposed.

4. The grasping treatment instrument of Claim 1,
wherein the second grasping piece includes a grasping surface which is opposed to the first grasping piece, and with which a treated target grasped between the first grasping piece and the second grasping piece is put in contact, and
the supported portion and the ring portion are located on the proximal side with respect to the grasping surface.

5. The grasping treatment instrument of Claim 1,
wherein an entirety of the ring portion is one piece.

6. The grasping treatment instrument of Claim 1,
wherein a distance from an outer edge, which is remotest from the longitudinal axis in a direction crossing the longitudinal axis in the movable member or the base member, in which the recess portion is provided, to the rotational axis is greater than a dimension of a cavity, which is located on an inner peripheral side of the ring portion, in the radiation direction of the base member.

7. The grasping treatment instrument of Claim 1,
wherein the first grasping piece includes a first grasping surface which is opposed to the second grasping piece, and a first groove, which is recessed from the first grasping surface, is formed in the first grasping piece,
the second grasping piece includes a second grasping surface which is opposed to the first grasping piece, and a second groove, which is recessed from the second grasping surface, is formed in the second grasping piece, and
the grasping treatment instrument further includes a cutter which is inserted in the first groove and the second groove, and is movable in the first groove and the second groove.

8. The grasping treatment instrument of Claim 7,
wherein a notch, in which the cutter is inserted, is formed on an inner peripheral surface of the ring portion.

9. The grasping treatment instrument of Claim 1,
wherein at least one of the first grasping piece and the second grasping piece includes at least one of an electrode configured to pass a high-frequency current through a treated target grasped between the first grasping piece and the second grasping piece by being supplied with electric energy, and a heating body configured to generate heat by being supplied with electric energy.

10. The grasping treatment instrument of Claim 1, further comprising a vibration transmitting member inserted through base member, and configured to transmit ultrasonic vibration from the proximal side to the distal side,
wherein the first grasping piece is formed of a projecting portion of the vibration transmitting member from the base member.
